# EUROPÄISCHE PATENTSCHRIFT

(11) **EP 1 291 039 B1**
(45) Veröffentlichungstag und Bekanntmachung des Hinweises auf die Patenterteilung: **14.06.2006**
(21) Anmeldenummer: 01121321.2
(22) Anmeldetag: 06.09.2001
(51) Int. Cl.: A61P 41/00, A61P 23/00, A61K 31/165

(54) **Verwendung eines Gemisches von Lidocain und Prilocain, sowie Mittel zur Tumeszenz-Lokalanästhesie**
Use of a mixture of lidocaine and prilocaine and composition for tumescent local anesthesia
Utilisation d'un mélange de lidocaine et prilocaine, et composition pour anesthésie locale tumescente

(43) Veröffentlichungstag der Anmeldung: 12.03.2003
(73) Patentinhaber: Friedrich, Wolfgang, Dr., 22303 Hamburg (DE)
(72) Erfinder: Friedrich, Wolfgang, Dr., 22303 Hamburg (DE); Schneider-Affeld, Frank Dr., Klinik Klosterstrasse, D-24536 Neumünster (DE)
(74) Vertreter: Fleck, Thomas

(56) Entgegenhaltungen:
- WO-A-91/07171
- US-A- 5 747 060
- US-A- 5 994 372
- BREUNINGER H ET AL: "Ropivacaine: an important anesthetic agent for slow infusion and other forms of tumescent anesthesia." DERMATOLOGIC SURGERY, (1999 OCT) 25 (10) 799-802. , XP001066248
- VAN AKEN H. ET AL: "[ Tumescent local anesthesia]." ANASTHESIOLOGIE UND INTENSIVMEDIZIN, (2000) 41/2 (114-115). , XP008001799

## Beschreibung

Die Erfindung betrifft die Verwendung eines Gemisches von Lidocain und Prilocain, sowie ein Mittel zur Tumeszenz-Lokalanästhesie.

Beide Wirkstoffe sind seit Jahrzehnten bekannt, siehe US 2,441,498 von 1943 bzw. GB 839 943 von 1957.

Die sogenannte Liposuktion oder Fettabsaugung ist eine Technik, unter Sog mit Hohlnadeln Fettgewebe aus der Körperoberfläche abzusaugen. Die Liposuktion erfolgte zunächst routinemäßig in Vollnarkose, an nicht vorbehandeltem Fettgewebe, mit großen Absaugkanülen und damit verbundener starker Gewebetraumatisierung und entsprechendem Blutverlust (Fischer A.; Fischer G: Revised techniques for cellulitis fat reduction in riding breechesdeformatity, Bull Int. Acad. Cosmet. Surg. 1; 40-41,1977).

Die Fettabsaugung dient der ästhetischen Konturierung vor allem in Bereich der Beine, des Bauches, der Hüften, der Kinn und Extremitätenregion. Neben den ästhetischen Indikationen wird sie auch bei benignen Fettgewebserkrankungen, die Lipodystrophysyndromen angewandt (Sattler G.;Hasche E; Rapprich S.; Mössler K; Hagedorn M;: Neue operative Behandlungsmöglichkeiten bei benignen Fettgewebserkrankungen (New approaches in benignen lipodystrophia) Zeitschrift für Hauterkrankungen, H + G 8 (72) 579-582 (1997)).

Nach dem sich die Methode der Tumeszenzlokalanästhesie in den 70er Jahren als trockene Absaugung etabliert hat, wurden in den 80er Jahren erste Versuche einer Nassabsaugung durchgeführt (Illouz Y: Body contouring by lipolysis: A 5 year experience with over 3000 cases Plast. Reconstr. Surg. 72; 511-524, 1983). Der amerikanische Dermatologe Jeff Klein stellte 1987 eine Tumeszenzlokalanästhesie (TLA), die in einigen Variationen grundlegend jedoch bis heute verwendet wird (Klein JA: Tumescent Techniques Chronicles. Dermatol. Surg. 21 ; 449-457, 1995, Collins PS; The Methodology of Liposuction Surgery Dermatologic Clinics - Vol.8, No3; 394-400, 1990). Dabei werden große Mengen physiologischer Kochsalzlösung mit einem Lokalanästhetikum vermischt, so daß eine Konzentration zwischen 0,05 und 0,1% zur Lokalanästhesie verwendet wird. Weitere Zusätze sind Adrenalin und Natriumbicarbonat sowie Triamcinolon Kristallsuspension. Der Adrenalinzusatz dient der Senkung der Blutungsneigung sowie der Resorptionsverzögerung der Tumeszenzflüssigkeit und des Lokalanästhetikums. Der Corticoidzusatz ist zur Verhinderung postoperativer Entzündungsreaktionen und Schwellungen gedacht, der Zusatz von Natriumbicarbonat soll eine Pufferung der Lösung erzielen, sowie das typische, ansonsten beim Injizieren von Lokalanästhetika verursachte Brennen verhindern. Als Standardlösungen sind im Stand der Technik angegeben:

Die Rezeptur der TLA nach Klein:

| | |
|---|---|
| Lidocain | 50,0 ( 1% ohne Adrenalin ) |
| Adrenalin | 1,0 ( 1: 1000 ) |
| Natriumbicarbonat | 12,5 ( 8,4 % ) |
| Triamcinolon-Acetonid | 1,0 ( 10 mg ) |
| NaCl 0,9 % | 1000,0 |

**Alternativ die Rezeptur nach Sattler :**

| | |
|---|---|
| Prilocain | 50,0 ( 1% ohne Adrenalin ) |
| Adrenalin | 1,0 ( 1 : 1000 ) |
| Natriumbicabonat | 6,0 (8,4 % ) |
| Triamcinolon-Acetonid | 1,0 (10 mg ) |
| Nacl 0,9% | 1000,0 |

Die vorbekannten Standardrezepturen der TLA werden weltweit seit Jahrzehnten jeweils mit nur einem Lokalanästhetikum durchgeführt, dies ist Lidocain z.B. in den USA und Prilocain z.B. in Deutschland. Beide Substanzen haben ihre spezifischen Nebenwirkungen, besonders in den für die TLA notwendigen Mengen. Diese sind für das Lidocain im wesentlichen zentralnervöser Natur, das Hauptrisiko von Prilocain besteht in einer Bindung an die roten Blutkörperchen, die damit zur Sauerstoffversorgung nicht zur Verfügung stehen. Dieses Phänomen wird als Methämoglobinämie bezeichnet. Unerfreulicherweise tritt die Methämoglobinämie mit einer Verzögerung von mehreren Stunden auf, die niedrigsten Sauerstoffdruckwerte im Blut werden etwa nach 10 bis 12 Stunden nach Infiltration gemessen. Dieser Zustand geht mit einer peripheren Cyanose, Schwindelgefühl und Kopfschmerzen einher und ist für den Patienten außerordentlich unangenehm. Darüber hinaus verdecken die Symptome der Methämoglobinämie möglicherweise das Vorliegen einer Lungenembolie. Aufgabe der Erfindung ist es, diese Nebenwirkungen, die weltweit in Kauf genommen werden, möglichst weitestgehend zu verringern. Generell besteht natürlich Bedarf, den Patientenkomfort zu erhöhen.

In überraschender Weise wird diese Aufgabe durch die Verwendung eines Gemisches von Lidocain und Prilocain zur Herstellung einer pharmazeutischen Zubereitung für die Tumeszenz-Lokalanästhesie mittels subkutaner Administration bei der Liposuktion und anderen chirurgischen Eingriffen am menschlichen Körper bzw. ein Mittel zur Tumeszenz-Lokalanästhesie, gekennzeichnet durch ein Gemisch der Wirkstoffe Lidocain und Prilocain oder deren Salzen, umfassend eine isotonische NaCl-Lösung, bei der beide Wirkstoffe in einem Gewichtsverhältnis von 75 : 25 bis 25 : 75 für die subkutane Administration bei der Liposuktion und anderen chirurgischen Eingriffen am menschlichen Körper vorliegen, gelöst.

Die fixe Kombination von Prilocain und Lidocain ist bereits aus der ausschließlich topischen transdermalen Anwendung für Haut- und Schleimhaut bekannt, siehe beispielsweise die US-A-5,332,576 oder WO 99/55287. Für den Mediziner oder Galeniker ist es selbstverständlich, daß sich aufgrund der transdermal topischen Anwendung keine Rückschlüsse auf ihre etwaige Anwendung im Rahmen der Tumeszenz-Lokalanästhesie ziehen lassen. Auch ist es für den Durchschnittsfachmann nicht naheliegend, sondern überraschend zu werten, daß die Lokalanästhesiekombination von Prilocain und Lidocain das Auftreten beispielsweise einer Methämoglobinämie nachweislich minimiert, was eine Toxizitätsstudie beweist.

Im folgenden wird die erfindungsgemäße Verwendung bzw. das Mittel anhand des Ablaufes ihres bzw. seines Einsatzes anhand mehrerer Ausführungsbeispiele zum besseren Verständnis der Erfindung beschrieben, ohne hierauf ausdrücklich beschränkt zu sein:

Es wird eine erfindungsgemäße Rezeptur der TLA-Lösung erstellt, die pro Liter NaCl 0,9 %, 25 ml 1% Lidocain ohne Adrenalin und 25 ml 1% Prilocain ohne Adrenalin beinhaltet. Die weiteren Bestandteile Adrenalin, Natriumhydrogencarbonat, Triamcinolon und NaCl 0,9% als Trägerlösung wurden unverändert, entsprechend der Rezepturen sowohl nach Klein als auch nach Sattler beibehalten. Eine Variation der Mischungsverhältnisse von 75 : 25 bis 25 : 75 werden jedoch als erfindungsgemäß angesehen. Die Lösung wird in der Regel frisch angesetzt, d.h. nur wenige Stunden vor dem geplanten Eingriff steril erstellt. Denkbar ist es jedoch auch, die Mischung als Fertigarzneimittel zum sofortigen Gebrauch ohne weitere Vorbereitungen zu stellen. Eine weitere Modifikation der Lösung könnte es sein, dem Gemisch noch 7,5 g Vitamin C beizumischen. Bisher wird das Vitamin C etwa 6 Stunden nach Abschluß der Liposuktion in Tumeszenzlokalanästhesie i.v. gegeben. Die klinischen Erfahrungen zeigen, daß Vitamin C vorteilhafterweise als Partner im Redoxsystem in der Lage ist, in einem geringen Maße ebenfalls die Methämoglobinämie zu mindern.

Die Ergebnisse einer Toxizitätsstudie an Ratten zeigen eine deutliche Reduktion der Komplikationen bei der Kombinationsanwendung von Prilocain und Lidocain im Vergleich zu Lidocain oder Prilocain allein.

Unter den vorliegenden Testbedingungen, beginnend mit der Zuführung als Einzeldosis subkutan in einer Kombination von 50% Lidocain und 50% Prilocain in einer Dosis von 107,5 mg Lidocain / kg + 107,5 mg Prilocain / kg an Ratten wurden reduzierte Motilität Ataxie, reduzierter Muskeltonus, Luftnot, klonische Krämpfe und Lateralpositionen beobachtet, jedoch nicht tonische Krämpfe und Tod, die wurden erst notiert ab 158 mg Lidocain + 158 mg Prilocain / kg Körpergewicht aufwärts. Zusätzliche Bauchlage trat auf bei Ratten, die mit 232 mg Lidocain und 232 mg Prilocain / kg Körpergewicht behandelt wurden.

Im Vergleich zur erfindungsgemäßen Verwendung wurden unter den vorgegebenen Testbedingungen, beginnend mit einer Administration als Einzeldosis subkutan von Lidocain in einer Dosis von 215 mg / kg Körpergewicht, an Ratten reduzierte Motilität, Ataxie, reduzierter Muskeltonus, Luftnot, klonische Krämpfe, Lateralposition und insbesondere schon Tod beobachtet. Tremor und abdominale Positionen wurden beobachtet von 316 mg Lidocain / kg Körpergewicht an aufwärts. Zusätzlich Tränenfluß trat auf bei Ratten, die mit 464 mg Lidocain / kg Körpergewicht behandelt wurden.

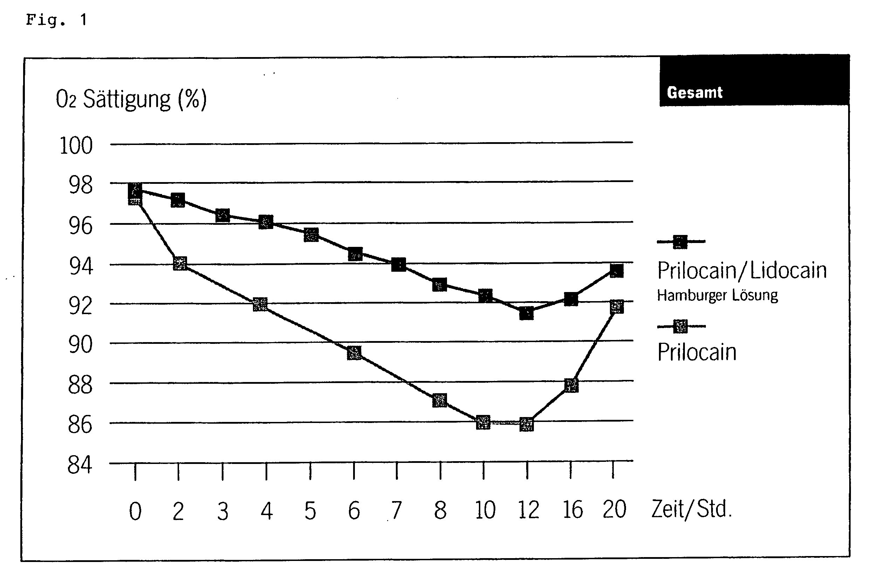

Erste klinische Erfahrungen zeigen, daß bei den mit den erfindungsgemäßen infiltrierten Patienten zur Liposuktion, die Sauerstoffsättigung, transcutan gemessen, bei einem Ausgangswert von 97 - 99% auf durchschnittlich maximal 89-92% abrutschte. Fig. 1 zeigt die graphische Darstellung der O₂ Sättigung bei einer Vielzahl (n=38) von Patienten bei Prilocain-Behandlung allein im Vergleich zum erfindungsgemäßen Mittel. Dies ist ein Bereich der für den Patienten sowohl medizinisch als auch psychisch gut zu verkraften ist, relevante Nebenwirkungen wie Kopfschmerzen, Müdigkeit, Schwindel, Vitationszustände wurden dabei nicht beobachtet. Bei der bisher verwendeten reinen Prilocainlösung kam es zu Sauerstoffentsättung bis 86% (im Einzelfall 80%), was mit einem erheblichen Beschwerdebild und teilweise einer stationären Überwachungsnotwendigkeit einhergehen, worauf erfindungsgemäß verzichtet werden kann. Diese ersten Daten belegen, daß mit der erfindungsgemäßen Verwendung, Mittel die Komplikationsrate erheblich absenken.

## Patentansprüche

1. Verwendung eines Gemisches von Lidocain und Prilocain zur Herstellung einer pharmazeutischen Zubereitung für die Tumeszenz-Lokalanästhesie mittels subkutaner Administration bei der Liposuktion und anderen chirurgischen Eingriffen am menschlichen Körper.

2. Verwendung des Gemisches nach Anspruch 1, **gekennzeichnet durch** den Einsatz von bis zu 10 1 0,9% NaCl-Lösung mit 250 ml 1% Lidocain und 250 ml 1% Prilocain pro Eingriff.

3. Mittel zur Tumeszenz-Lokalanästhesie, **gekennzeichnet durch** ein Gemisch der Wirkstoffe Lidocain und Prilocain oder deren Salzen, umfassend eine isotonische NaCl-Lösung, bei der beide Wirkstoffe in einem Gewichtsverhältnis von 75 : 25 bis 25 : 75 für die subkutane Administration bei der Liposuktion und anderen chirurgischen Eingriffen am menschlichen Körper vorliegen.

4. Mittel nach Anspruch 3, **gekennzeichnet durch** eine Kombination beider Wirkstoffe in einem Gewichtsverhältnis von 50 : 50.

5. Mittel nach Anspruch 4, **dadurch gekennzeichnet, daß** es aus Lidocain HCl und Prilocain HCl besteht.

6. Mittel nach Anspruch 3 bis 5, **dadurch gekennzeichnet, daß** die isotonischen NaCl-Lösungen beider Wirkstoffe unmittelbar vor dem Gebrauch zusammengegossen werden, oder als lager- und gebrauchsfertige Arzneimittellösung zur Verfügung gestellt wird.

7. Mittel nach einem oder mehreren der vorstehenden Ansprüche, **gekennzeichnet durch** einen oder mehrere der folgenden Zusatzstoffe:
Adrenalin,
Natriumhydrogencarbonat,
Triancinolon-Acetonid,
Ascorbinsäure.

## Claims

1. Use of a mixture of lidocaine and prillocaine for preparing a pharmaceutical formulation for tumescence local anesthesia by means of subcutaneous administration in connection with liposuction and other surgical interventions on the human body.

2. Use of the mixture according to claim 1, **characterized by** the use of up to 10 1 0.9% NaCl solution with 25 ml 1% lidocaine and 250 ml 1% prillocaine per intervention.

3. Agent for tumescence local anesthesia, **characterized by** a mixture of the active ingredients lidocaine and prillocaine or their salts, comprising an isotonic NaCl solution, in which the two active ingredients are present in a weight ratio of 75:25 to 25:75 for subcutaneous administration in connection with liposuction and other surgical interventions on the human body.

4. Agent according to claim 3, **characterized by** a combination of both active ingredients in a weight ratio of 50:50.

5. Agent according to claim 4, **characterized in that** it comprises lidocaine HCl and prillocaine HCl.

6. Agent according to claims 3 to 5, **characterized in that** the isotonic NaCl solutions of both active ingredients are poured together directly prior to use, or are made available as a ready to store and use medicinal solution.

7. Agent according to one or more of the preceding claims, **characterized by** one or more of the following additives: adrenaline, sodium hydrogen carbonate, triamcinolone-acetonide and ascorbic acid.

## Revendications

1. Utilisation d'un mélange de lidocaïne et de prilocaïne pour la préparation d'une formulation pharmaceutique pour l'anesthésie locale d'une tumescence par administration sous-cutanée, dans la liposuccion et d'autres interventions chirurgicales sur le corps humain.

2. Utilisation du mélange suivant la revendication 1, **caractérisée par** le recours à jusqu'à 10 litres de solution à 0,9 % de NaCl avec 250 ml de lidocaine à 1 % et de 250 ml de prilocaine à 1 % par intervention.

3. Composition destinée à l'anesthésie locale d'une tumescence, **caractérisée par** un mélange des substances actives lidocaïne et prilocaine ou leurs sels, comprenant une solution isotonique de NaCl dans laquelle les deux substances actives sont présentes dans un rapport en poids de 75:25 à 25:75 pour l'administration sous-cutanée dans la liposuccion et dans d'autres interventions chirurgicales sur le corps humain.

4. Composition suivant la revendication 3, **caractérisée par** une combinaison des deux substances actives dans un rapport en poids de 50:50.

5. Composition suivant la revendication 4, **caractérisée en ce qu'**elle est constituée de chlorhydrate de lidocaïne et de chlorhydrate de prilocaïne.

6. Composition suivant les revendications 3 à 5, **caractérisée en ce que** les solutions isotoniques de NaCl des deux substances actives sont réunies par coulée juste avant leur utilisation ou sont mises à disposition sous forme de solutions médicamenteuses prêtes au stockage et à l'emploi.

7. Composition suivant une ou plusieurs des revendications précédentes, **caractérisée par** la présence d'un ou plusieurs des additifs suivants :
adrénaline,
hydrogénocarbonate de sodium,
triancinolone-acétonide,
acide ascorbique.
